# EUROPEAN PATENT SPECIFICATION

(11) **EP 3 255 430 B1**
(45) Date of publication and mention of the grant of the patent: **25.03.2020**
(21) Application number: 17175053.2
(22) Date of filing: 08.06.2017
(51) Int. Cl.: G01N 33/50

(54) **TOXICANT ASSAYS FOR COSMETIC PRODUCTS USING TELEOST EMBRYOS**
GIFTSTOFFASSAYS FÜR KOSMETISCHE PRODUKTE MITTLES TELEOST EMBYONEN
DOSAGES DE SUBSTANCES TOXIQUES POUR PRODUITS COSMÉTIQUES EMPLOYANT D'EMBRYONS TELEOST

(30) Priority: 08.06.2016 US 201662347132 P
(43) Date of publication of application: 13.12.2017
(73) Proprietor: Vitargent (International) Biotechnology Limited, Shatin, N.T. (HK)
(72) Inventor: CHEN, Xueping, Shatin, N.T. (HK); CHEN, Zixiang, Shatin, N.T. (HK); TAO, Wai Leung, Shatin, N.T. (HK)
(74) Representative: J A Kemp LLP

(56) References cited:
- EP-A2- 2 368 430
- US-A1- 2004 147 030
- XUEPING CHEN ET AL: "Toxicity and Estrogenic Endocrine Disrupting Activity of Phthalates and Their Mixtures", INTERNATIONAL JOURNAL OF ENVIRONMENTAL RESEARCH AND PUBLIC HEALTH, vol. 11, no. 3, 14 March 2014 (2014-03-14) , pages 3156-3168, XP055317199, DOI: 10.3390/ijerph110303156
- BRAUNBECK THOMAS ET AL: "The fish embryo test (FET): origin, applications, and future", ENVIRONMENTAL SCIENCE AND POLLUTION RESEARCH INTERNATIONAL, ECOMED, LANDSBERG, DE, vol. 22, no. 21, 15 November 2014 (2014-11-15), pages 16247-16261, XP035576755, ISSN: 0944-1344, DOI: 10.1007/S11356-014-3814-7 [retrieved on 2014-11-15]
- CHEN H ET AL: "Generation of a fluorescent transgenic zebrafish for detection of environmental estrogens", AQUATIC TOXICOLOGY, ELSEVIER, AMSTERDAM, NL, vol. 96, no. 1, 21 January 2010 (2010-01-21), pages 53-61, XP026858863, ISSN: 0166-445X [retrieved on 2009-10-01]
- VERA DELOV ET AL: "Transgenic fluorescent zebrafish Tg(fli1:EGFP)y1 for the identification of vasotoxicity within the zFET", AQUATIC TOXICOLOGY, vol. 150, 1 May 2014 (2014-05-01), pages 189-200, XP055420207, AMSTERDAM, NL ISSN: 0166-445X, DOI: 10.1016/j.aquatox.2014.03.010
- Kouichi Goka: "Embryotoxicity of Zinc Pyrithione, An Antidandru? Chemical, in Fish", Environmental research, 1 January 1999 (1999-01-01), pages 81-83, XP055317787, Retrieved from the Internet: URL:http://ac.els-cdn.com/S001393519893944 5/1-s2.0-S0013935198939445-main.pdf?_tid=d 0a507e0-a672-11e6-9614-00000aab0f01&acdnat =1478692453_9d379bb8240a2b9c472e2f3d2de1a9 88 [retrieved on 2016-11-09]

## Description

### 1. FIELD OF INVENTION

The invention relates to methods for determining whether a toxicant is present in a cosmetic product. Particularly, the invention relates to methods of using teleost embryo in assays to determine the presence of a toxicant in a cosmetic product.

### 2. BACKGROUND

With the rise in modernization and globalization, consumer products, such as cosmetics, can go through many processes in which potentially toxic chemicals can enter the products before reaching the consumer. The US Environmental Protection Agency tracks or regulates more than 100,000 chemicals (Substance Registry Services Fact Sheet, available at
ofmpub.epa.gov/sor_internet/registry/substreg/educationalresources), and the toxicity of many of these chemicals have not been well studied, especially their total biological toxicity when combined with other chemicals. Ensuring the safety of cosmetic products is a great challenge to the modern testing industry given the sheer number of potentially toxic chemicals and chemical combinations that can find their way into cosmetic products.

To date, toxicity testing of cosmetic products still largely relies on chemical-specific tests, especially chemical analysis. For example, a review article relates to an overall view of the state-of-the-art and the technical knowhow concerning the extraction and chromatographic techniques for the determination of allowed ingredients in cosmetic products (Analytica Chimica Acta 915, (2016), pp. 1-26). While chemical-specific tests can be sensitive and precise, they can fail to detect unknown toxicants that are not intended to be specifically tested; this can allow unanticipated toxicants to go undetected. Even in cases where the chemical composition of a sample is known in detail, its effective toxicity cannot necessarily be reliably predicted due to the lack of knowledge concerning the effects of chemical mixtures. Practical experience with studies has shown that chemical-specific measurements identify true toxicity in unknown samples only about 20% of the time, which means up to 80% toxicants are unidentified.

Thus, new methods for determining whether a toxicant is present in a cosmetic product are needed. For example, an article provides early-life-stage toxicity tests of zinc pyrithione (Zpt) and commercial shampoos containing or not containing Zpt are performed on zebra fish and Japanese Medaka (Environmental Research Section A 81, 81-83, 1999). US 2013/152222 relates to transgenic fishes and their use in, inter alia, detecting estrogenic and anti-estrogenic compounds, monitoring estrogen-like activity in the environment, and elucidating liver regeneration. Other methods are disclosed by, for example, EP2368430A which discloses transgenic fish and uses thereof. Chen et al. (Int J Environ Res Public Health. 2014 Mar 14;11(3):3156-68) discloses the toxicity and estrogenic endocrine disrupting activity of phthalates and their mixtures. Thomas et al. (Environ Sci Pollut Res Int. 2015 Nov;22(21):16247-61) discloses the fish embryo test (FET): origin, applications, and future. Chen et al. (Aquat Toxicol. 2010 Jan 21;96(1):53-61) discloses a generation of a fluorescent transgenic zebrafish for detection of environmental estrogens. US 2004/147030 discloses transgenic animals for monitoring water quality. Delov et al. (Aquat Toxicol. 2014 May; 150:189-200) discloses transgenic fluorescent zebrafish Tg (fli1:EGFP)y for the identification of vasotoxicity within the zFET. Goka et al. (Environ Res. 1999 Jul;81(1):81-3) discloses the embryotoxicity of zinc pyrithione, an antidandruff chemical, in fish. However, there is a need to develop a bioassay to detect toxicants in a sample.

### 3. SUMMARY

The disclosure provides methods of determining whether a toxicant is present in a cosmetic product, which comprise contacting a teleost embryo with a sample of the cosmetic product or an extract from a sample of a cosmetic product and determining whether the sample or the extract exerts a toxicity effect on the embryo, where a toxicity effect on the embryo is indicative of the presence of a toxicant in the cosmetic product. Exemplary cosmetic products that can be tested using the methods of the disclosure are described in Section 0. The extract may comprise an organic solvent extract (e.g., a methanol extract). The extract may comprise an organic and inorganic solvent extract (*e.g.,* a methanol and water extract). Methods for preparing an extract from a cosmetic product for toxicant testing are described in Sections 0 and 0 and in numbered embodiments 9 to 21 below.

In some embodiments, the testing methods of the disclosure comprise determining whether the sample or extract exerts a toxicity effect on teleost embryos, such as an acute effect (*e.g.,* malformation or death) or a specific effect (*e.g.,* estrogen activity disruption). In some embodiments, the testing methods comprise contacting a teleost embryo with an organic solvent extract or organic and inorganic solvent extract that is obtainable or obtained by a process as described in Section 0, Section 0 or one of numbered embodiments 9 to 21 below. Exemplary methods of determining whether a toxicant is present in a cosmetic product are described in Sections 0 and numbered embodiments to 64 below. In some embodiments, the teleost is a medaka or a zebrafish embryo (as described in Sections 0 and 0 and can in some embodiments be transgenic. Acute and specific toxicity effects that can be determined using the methods of the disclosure are described in Section 0 and 0, respectively. Exemplary acute toxicity effects include mortality, and malformation, and exemplary specific toxicity effects include estrogen activity disruption, androgen activity disruption, xenobiotic effect, cardiotoxicity effect, and hepatotoxicity effect. The sample testing methods provided by the disclosure can be used, for example, to evaluate the total biological toxicity effects of samples of cosmetic products or extracts from cosmetic products. The methods of the disclosure can be applied in a high throughput manner to test large numbers of samples, providing, for example, a means to determine the biological safety of a large number of cosmetic products.

### 4. DETAILED DESCRIPTION

### 4.1. Overview

The invention creates a biological assay method for determining toxicity profile in a cosmetic product sample. Significantly different from a chemical assay that is directed to detection of a specific toxicant(s) but not unspecified toxicants, the biological assay of the invention obtains an overall toxicity profile in the sample and can be used an index of toxicity of a sample. In contrast to the chemical assay, no toxic substance is added to the sample during sample pretreatment process used in the method of the invention and agents used in the method will not react with the sample and change sample toxicity. Accordingly, the invention provides a method of determining an overall toxicity in a cosmetic product. Accordingly, the present invention provides a method of determining the overall toxicity in a cosmetic product, comprising:
a) combining a solvent and a sample of the cosmetic product to obtain a solvent extract with an overall toxicity;
b) contacting a teleost embryo with the solvent extract of a); and
c) determining whether the extract exerts a toxicity effect on the embryo;
wherein a toxicity effect on the embryo shows an overall toxicity of the cosmetic product; and wherein the extract is (i) an organic solvent extract or (ii) an organic and inorganic solvent extract; wherein the organic solvent is methanol, DMSO, acetonitrile or acetone, and wherein the inorganic solvent is water; wherein when the sample of the cosmetic product is a liquid, the volume of the solvent is about 1.5 to about 10 times the volume of the sample; or when the sample of the cosmetic product is a solid or semi-solid, the volume of the solvent is about 1.5 to about 10 times the weight of the sample.

In one aspect, the disclosure provides a method of determining an overall toxicity in a cosmetic product, comprising:
a) combining a solvent and the cosmetic product to obtain a solvent extract with an overall toxicity;
b) contacting a teleost embryo with the solvent extract of a); and
c) determining whether the extract exerts a toxicity effect on the embryo;
wherein a toxicity effect on the embryo shows an overall toxicity of the cosmetic product.

The disclosure provides methods of determining whether a toxicant is present in a cosmetic product. The methods comprise contacting a teleost embryo with a sample of the cosmetic product or an extract from a sample of the cosmetic product and determining whether the sample or extract exerts a toxicity effect on the embryo, where a toxicity effect on the embryo indicates the presence of a toxicant in the cosmetic product. Advantageously, the methods of the disclosure can be used to monitor the total biological toxicity of a cosmetic, in contrast to chemical specific tests which generally detect the presence of only one type of toxicant or one group of toxicants. Exemplary cosmetic products that can be tested using the methods of the disclosure are described in Section 0. Processes for preparing extracts from samples of cosmetic products are described in detail in Sections 0 and 4.4, and methods of determining whether a toxicant is present in a cosmetic product sample or cosmetic product extract are described in Section 0.

### 4.2. Cosmetic products

The cosmetic product can be any substance or mixture intended to be placed in contact with the various external parts of a human or animal body (epidermis, hair system, nails, lips and external genital organs) or with the teeth and the mucous membranes of the oral cavity with a view exclusively or mainly to cleaning them, perfuming them, changing their appearance and/or correcting body odors and/or protecting them or keeping them in good condition. In some embodiments, the cosmetic product is a product that is intended to be applied to a human body.

Exemplary categories of cosmetic products include, but are not limited to, creams, emulsions, lotions, gels and oils for the skin (e.g., hands, face, feet, etc.); face masks; tinted bases (*e.g.,* liquids, pastes, powders); make-up powders, after-bath powders, hygienic powders, etc.; toilet soaps, deodorant soaps, etc.; perfumes, toilet waters and eau de Cologne; bath and shower mixtures (*e.g.,* salts, foams, oils, gels, etc.); depilatories; deodorants and anti-perspirants; hair care products (*e.g.,* hair tints and bleaches, products for waving, straightening and fixing, setting products, cleansing products (*e.g.,* lotions, powders, shampoos), conditioning products (*e.g.,* lotions, creams, oils), hairdressing products (*e.g.,* lotions, lacquers, brilliantines)); shaving products (*e.g.,* creams, foams, lotions, etc.); products for making up and removing make-up from the face and the eyes (e.g., make-up remover); products intended for application to the lips; products for care of the teeth and the mouth; products for nail care and make-up; products for external intimate hygiene; sunbathing products; products for tanning without sun; skin-whitening products; and anti-wrinkle products.

Exemplary cosmetics include mask, perfume, antiperspirant, deodorant, toothpaste, shampoo, essential oil, make-up remover, cleansing product, hair dye, foundation, concealer, sunscreen, moisturizer, anti-wrinkle cream, eyeshadow, eyeliner, mascara, blush, lipstick, lip gloss, nail polish, serum, eye cream, night cream, day cream, BB cream, night lotion, day lotion, hand cream, neck cream, anti-aging cream, body wash, shampoo, and hair conditioner. In some embodiments, the cosmetic is a foundation. In some embodiments, the cosmetic is a concealer. In some embodiments, the cosmetic is a sunscreen. In some embodiments, the cosmetic is a moisturizer. In some embodiments, the cosmetic is an anti-wrinkle cream. In some embodiments, the cosmetic is an eyeshadow. In some embodiments, the cosmetic is an eyeliner. In some embodiments, the cosmetic is a mascara. In some embodiments, the cosmetic is a blush. In some embodiments, the cosmetic is a lipstick. In some embodiments, the cosmetic is a lip gloss. In some embodiments, the cosmetic is a nail polish. In some embodiments, the cosmetic is a serum. In some embodiments, the cosmetic is an eye cream. In some embodiments, the cosmetic is a night cream. In some embodiments, the cosmetic is a day cream. In some embodiments, the cosmetic is BB cream. In some embodiments, the cosmetic is night lotion. In some embodiments, the cosmetic is a day lotion. In some embodiments, the cosmetic is a hand cream. In some embodiments, the cosmetic is a neck cream. In some embodiments, the cosmetic is an anti-aging cream. In some embodiments, the cosmetic is body wash. In some embodiments, the cosmetic is make-up remover. In some embodiments, the cosmetic is hair dye. In some embodiments, the cosmetic is shampoo. In some embodiments, the cosmetic is a hair conditioner.

Samples of cosmetic products that can be tested using the methods of the disclosure include the entire cosmetic product (*e.g.,* the entire contents of a tube of face cream) or a portion thereof. The term "sample" also includes cosmetic product samples that have been diluted in a solvent (*e.g.,* a sample of a water miscible cosmetic diluted in water or a teleost culture medium).

### 4.3. Cosmetic product extracts

Extracts that can be used in the toxicity testing methods of the disclosure can be prepared from samples of cosmetic products, such as those described previously in Section 0. Extracts that can be used in the toxicity testing methods of the disclosure are preferably organic solvent extracts or organic and inorganic solvent extracts. The term "organic solvent" when used in connection with the term "organic solvent extract" refers to the particular organic solvent or mixture of organic solvents used to extract compounds from a sample of a cosmetic product and does not necessarily refer to the solvent in which the extract may be dissolved in at any given time. For example, an extract prepared by extracting compounds from a sample using methanol remains a methanol extract even in instances in which the extract is processed to remove the methanol following extraction. Thus, for example, a methanol extract that has been dried to remove the methanol and subsequently redissolved or resuspended in another solvent (such as dimethyl sulfoxide) remains a methanol extract even though the extract in its present state contains another solvent other than methanol. Similarly, the terms "organic solvent" and "inorganic solvent" when used in connection with the term "organic solvent and inorganic solvent extract" refer to the organic and inorganic solvents used to extract compounds from a sample of a cosmetic product.

Organic solvents that can be used to make an organic solvent extract include methanol, dimethyl sulfoxide (DMSO), acetonitrile, acetone, toluene, diethyl ether, dichloromethane, chloroform, hexane, and mixtures thereof. Preferred organic solvents include organic solvents capable of dissolving polar and nonpolar organic molecules, such as methanol and DMSO. An exemplary inorganic solvent is water. In some embodiments, methanol is used to make an organic solvent extract. In other embodiments, DMSO is used to make an organic solvent extract. In other embodiments, a mixture of methanol and DMSO is used to make an organic solvent extract. In some embodiments, a mixture of methanol and water is used to make an organic and inorganic solvent extract. In some embodiments, a mixture of DMSO and water is used to make an organic and inorganic solvent extract.

Organic solvent extracts can be obtained by a process comprising combining a sample of the cosmetic product and an organic solvent (e.g., methanol) to form a mixture, mixing the mixture, separating a phase containing the organic solvent from the mixture, and recovering the extract. The volume of the organic solvent can be selected or varied based upon the amount and/or nature of the sample (*e.g.,* the consistency of the sample). The volume of organic solvent combined with the sample to form the mixture can be, for example, about 1.5 to about 10 times the weight or volume of the sample. That is, when the sample is a liquid, the volume of the solvent is about 1.5 to about 10 times the volume of the sample; when the sample is a solid or semi-solid, the volume of the solvent is about 1.5 to about 10 times the weight of the sample (*e.g.,* about 1.5 to about 8 times, about 2 to about 5 times, about 1.5 times to about 3 times, about 2 times to about 4 times, or about 2 times to about 4 times the weight or volume of the sample). In some embodiments, the volume of the organic solvent (*e.g.,* methanol) is at least 3 times the weight or volume of the sample. Higher organic solvent to sample ratios can in some instances allow for higher amounts of extracted toxicants in contrast to lower organic solvent to sample ratios; however, higher organic solvent to sample ratios result in more dilute extracts which may, depending on how dilute the extract is, require concentration prior to testing the extract in a toxicity assay.

Mixing the mixture can be performed, for example, by shaking the mixture, vortexing the mixture, sonicating the mixture, or a combination thereof. In some embodiments, mixing comprises vortexing and sonicating the mixture.

Separating a phase containing the organic solvent from the mixture can comprise centrifuging the mixture to separate the phase containing the organic solvent from the mixture. Alternatively, the phases can be separated under the force of gravity, although separating a mixture under the force of gravity may take longer to complete compared to separating the mixture using centrifugation. For cosmetics that contain solid particles (*e.g.,* glitter or mineral particles) which are not completely soluble or are insoluble in the organic solvent, filtering can be used to separate a phase containing the organic solvent from the mixture (*e.g.,* using gravity, centrifugal, or vacuum filtration).

Following separation, the organic solvent extract can be recovered, for example, by pipetting the phase containing the organic solvent away from the other phases, decanting the separated phases, or separating the phases using a separatory funnel (*e.g.,* when centrifugation is not used to separate the phases).

Processes for obtaining an organic solvent extract can include the use of one or more salts and/or sugars to aid in the extraction of toxicants from the sample of the cosmetic product to the organic solvent. For example, forming the mixture can comprise combining the sample of the cosmetic product with the organic solvent and with (i) a salt (*e.g.,* sodium chloride, magnesium sulfate, calcium chloride, magnesium chloride, sodium acetate, ammonium acetate, anhydrous sodium sulfate, anhydrous magnesium sulfate, anhydrous calcium chloride, anhydrous calcium sulfate, or a combination thereof), and/or (ii) a sugar (*e.g.,* a monosaccharide and/or disaccharide, such as glucose, xylose, arabinose, fructose, maltose, sucrose and mixtures thereof). In some embodiments, the salt is sodium chloride. In some embodiments, the sugar is sucrose. The use of an anhydrous salt can help to absorb water that may be present in the mixture and can be used to provide a dehydrated organic solvent extract. In the context of this disclosure, and unless required otherwise by context, a salt that is not specifically identified as being in hydrated or anhydrous form encompasses both hydrated and anhydrous forms of the salt. For example, "calcium chloride" encompasses anhydrous and hydrated forms of calcium chloride (*i.e.,* CaCl₂(H₂O)ₓ, where x = 0, 1, 2, 4, or 6). The salt and/or sugar can also be added to a preformed mixture of the sample of the cosmetic product and the organic solvent, for example until the mixture is saturated with the salt and/or sugar (*e.g.,* as indicated by the observance of salt or sugar crystals within the mixture that do not dissolve). A phase containing the organic solvent can then be separated from the mixture, and the organic solvent extract can be recovered, for example as described in the preceding paragraphs.

Processes for obtaining an organic solvent extract can also include a dehydration step and/or a delipidation step. The dehydration step can comprise combining the organic solvent extract with one or more anhydrous salts (*e.g.,* one or more of the anhydrous salt described in the preceding paragraph), optionally mixing the mixture, and then separating a phase containing the organic solvent from the mixture. The delipidation step can comprise washing the organic solvent extract with at least once (*e.g.,* once, twice, or three times) with a non-polar solvent. For example, C₅-C₈ alkanes (*e.g., n*-pentane, *n*-hexane, *n*-heptane, or *n*-octane) can be used. The solvent used for delipidation may comprise hexane. As used herein, "hexane" refers to n-hexane. As an alternative to performing an organic solvent extraction followed by a wash with a nonpolar solvent as described above, a delipidated organic solvent extract can be obtained by using hexane as the solvent to extract compounds from the cosmetic product and then subjecting the hexane extract to solvent extraction using a polar organic solvent such as acetonitrile.

In some embodiments, when the organic solvent is methanol or DMSO, the steps described in the two preceding paragraphs are not performed (*i.e.,* a salt and/or sugar is not used in the process, and the dehydration and delipidation steps described in this paragraph are not performed). In some embodiments, some or all of the steps described in the preceding two paragraphs are performed when the organic solvent is acetonitrile, acetone, toluene, diethyl ether, dichloromethane, chloroform, hexane, and mixtures thereof. An exemplary extraction protocol that includes steps described in the two preceding paragraphs is described below in Section 4.4.

The recovered organic solvent extract (for example, prepared according to a process described in any of the preceding paragraphs) can be further processed, for example, to concentrate the extract, to remove some or all of the organic solvent from the extract, or dilute the extract in a solvent appropriate for use in a toxicity assay. Organic solvent extracts can be concentrated by, for example, partially drying the extract under a stream of nitrogen or using a rotary evaporator. Organic solvent extracts can be completely dried to remove the organic solvent by, for example, drying the extract under a stream of nitrogen or using a rotary evaporator. Following drying, the organic solvent extract can then be redissolved or suspended in a second organic solvent. In some embodiments, the second organic solvent is a solvent that is appropriate for use in a toxicity assay as described in Section 0. Exemplary second organic solvents include methanol, dimethyl sulfoxide, and mixtures thereof.

When the organic solvent used in the extraction is appropriate for use in a toxicity assay, it is not necessary to dry the extract and redissolve or suspend the extract in another solvent. Instead, the extract can, for example, be concentrated and then diluted in another solvent, diluted in another solvent without being concentrated, or used directly in the assay without being concentrated or diluted. In some embodiments, the extract is neither dried nor concentrated prior to use in a toxicity assay. For example, an organic solvent extract made using methanol as the organic solvent can be used directly in a toxicity assay or can be diluted prior to use in the assay without being concentrated prior to dilution. Solvents that can be used to dilute an organic solvent extract prior to use in a toxicity assay include water and teleost culture media.

Organic and inorganic solvent extracts can be prepared similarly to organic solvent extracts except that a combination of an organic and inorganic solvent is used to prepare the extract instead of an organic solvent. In some embodiments, the organic and inorganic solvents are used in a ratio of 3:7 to 7:3 (*e.g.,* 3:7 to 7:3, 4:6 to 6:4, or 1:1). In some embodiments, the ratio is 4:6 or 6:4. In some embodiments, the organic solvent is methanol and the inorganic solvent is water. In some embodiments, the ratio of methanol to water is 3:7, 4:6, 1 :1, 6:4, or 7:3. In some embodiments, the ratio of methanol to water is 4:6. In other embodiments, the ratio of methanol to water is 6:4.

Organic and inorganic solvent extracts can be obtained by a process comprising combining a sample of the cosmetic product, an organic solvent (*e.g.,* methanol) and an inorganic solvent (*e.g.,* water) to form a mixture, mixing the mixture, separating a phase containing the organic solvent and inorganic solvent from the mixture, and recovering the extract. The total volume of the organic solvent and inorganic solvent (*i.e.,* the total solvent volume) can be selected or varied based upon the amount and/or nature of the sample (*e.g.,* the consistency of the sample). The total solvent volume can be, for example, about 1.5 to about 10 times the weight or volume of the sample (*e.g.,* about 1.5 to about 8 times, about 2 to about 5 times, about 1.5 times to about 3 times, about 2 times to about 4 times, or about 2 times to about 4 times the weight or volume of the sample). In some embodiments, the total solvent volume is at least 3 times the weight of the sample.

The mixing can be performed, for example, by shaking the mixture, vortexing the mixture, sonicating the mixture, or a combination thereof. In some embodiments, mixing comprises vortexing and sonicating the mixture.

Separating a phase containing the organic solvent and inorganic solvent from the mixture can comprise centrifuging the mixture to separate the phase containing the organic solvent and inorganic solvent from the mixture. Alternatively, the phases can be separated under the force of gravity, although separating a mixture under the force of gravity may take longer to complete compared to separating the mixture using centrifugation. For cosmetics that contain solid particles and which are not completely soluble or are insoluble in the organic solvent and inorganic solvent mixture, filtering can be used to separate a phase containing the organic solvent and organic solvent from the mixture (*e.g.,* using gravity, centrifugal, or vacuum filtration).

Following separation, the organic and inorganic solvent extract can be recovered, for example, by pipetting the phase containing the organic and inorganic solvent away from the other phases, decanting the separated phases, or separating the phases using a separatory funnel (*e.g.,* when centrifugation is not used to separate the phases).

The recovered organic and inorganic solvent extract can be further processed, for example, to concentrate the extract, to remove some or all of the organic and inorganic solvents from the extract, or dilute the extract in a solvent appropriate for use in a toxicity assay. Organic and inorganic solvent extracts can be concentrated by, for example, partially drying the extract under a stream of nitrogen or using a rotary evaporator. Organic and inorganic solvent extracts can be completely dried to remove the organic and inorganic solvent by, for example, drying the extract under a stream of nitrogen or using a rotary evaporator. Following drying, the organic and inorganic solvent extract can then be redissolved or suspended in a second organic solvent. In some embodiments, the second organic solvent is a solvent that is appropriate for use in a toxicity assay as described in Section 0. Exemplary second organic solvents include methanol, dimethyl sulfoxide, and mixtures thereof.

When the organic and inorganic solvents used in the extraction are both appropriate for use in a toxicity assay, it is not necessary to dry the extract and redissolve or suspend the extract in another solvent. Instead, the extract can, for example, be concentrated and then diluted in another solvent, diluted in another solvent without being concentrated, or used directly in the assay without being concentrated or diluted. In some embodiments, the extract is neither dried nor concentrated prior to use in a toxicity assay. For example, an organic and inorganic solvent extract made using methanol as the organic solvent and water as the inorganic solvent can be used directly in a toxicity assay or can be diluted prior to use in the assay without being concentrated prior to dilution. Solvents that can be used to dilute an organic and inorganic solvent extract prior to use in a toxicity assay include water and teleost cell culture media.

### 4.4. Exemplary extraction protocols

The following protocol is an exemplary protocol that can be used to prepare a methanol extract from a cosmetic product, for example from a non-water miscible cosmetic product.
1) combine a sample of the cosmetic product with an amount of methanol to form a mixture;
2) mix the mixture, optionally by vortexing and sonicating the mixture;
3) centrifuge the mixture; and
4) separate the supernatant from the mixture, thereby obtaining a methanol extract.

The methanol extract obtained by the above-described protocol can be directly used in a toxicity assay or can be diluted with a solvent (such as water or a teleost culture medium) without a concentration or solvent removal step.

The following protocol is an exemplary protocol for preparing a methanol and water extract from cosmetic product, for example from a non-water miscible cosmetic product.
1) combine a sample of the cosmetic with an amount of methanol and an amount of water to form a mixture;
2) mix the mixture, optionally by vortexing and sonicating the mixture;
3) centrifuge the mixture; and
4) separate the supernatant from the mixture, thereby obtaining a methanol and water extract.

The methanol extract obtained by the above-described protocol can be directly used in a toxicity assay or can be diluted with a solvent (such as water or a teleost culture medium) without a concentration or solvent removal step.

The following protocol is an exemplary protocol for preparing an acetonitrile extract from a cosmetic product, for example from a non-water miscible cosmetic product.
1) combine a sample of the cosmetic product with an amount of acetonitrile to form a mixture, and optionally mix the mixture;
2) add sodium chloride to the mixture until saturation, and optionally mix the mixture;
3) separate a phase containing acetonitrile from the mixture (*e.g.,* by centrifugation) to obtain an acetonitrile extract;
4) add anhydrous sodium sulfate to the acetonitrile extract until saturation to form a second mixture, and optionally mix the second mixture;
5) separate a phase containing acetonitrile from the second mixture (*e.g.,* by centrifugation) to obtain a dehydrated acetonitrile extract;
6) wash the dehydrated acetonitrile extract with hexane, and optionally repeat once to obtain a dehydrated and delipidated acetonitrile extract; and
7) remove the acetonitrile from the dehydrated and delipidated acetonitrile extract and redissolve the dehydrated and delipidated acetonitrile extract in methanol.

### 4.5. Testing for toxicity effect

Teleost embryos are an effective *in vivo* model system to screen/identify the biological effects, *e.g.,* toxicity effects, of a test sample, and the adverse effects identified using fish (*e.g.,* zebrafish and medaka fish) embryos is predictable to that of human beings. Fish embryos are not defined as protected animals under European legislation can be used as animal alternatives (Directive 2010/63/EU; Halder et al., 2010, Integrated Environmental Assessment Management. 6:484-491).

The screening assays of the disclosure entail contacting a teleost embryo with a sample of a cosmetic product or an extract from a sample of the cosmetic product and determining whether the extract exerts a toxicity effect on the embryo. A sample of the cosmetic product can be used in a toxicity assay without extraction, for example, if the cosmetic is water miscible. In some embodiments, a water miscible cosmetic is diluted, for example, in water or a teleost culture medium, and then used in a screening assay (*e.g.,* diluted 10 to 200 fold). For non-water miscible cosmetics, organic solvent (*e.g.,* methanol) or organic and inorganic solvent *(e.g.,* methanol and water) extracts are preferably used.

The teleost embryos that can be used in a screening assay of the disclosure can be of various freshwater, brackish water, or saltwater (marine water) species of fish, including, without limitation, fish of the *Oryzias* genus, the *Danio* genus and the *Pimephales* genus. Fish in the *Oryzias* genus belong to the *Adrianichthyidae* family and include, for example, *Oryzias melastigma* (alternative name *Oryzias dancena*) (marine or brackish medaka), *Oryzias latipes* (Japanese medaka), *Oryzias celebensis, Oryzias marmoratus, Oryzias matanensis, Oryzias nigrimas* (black buntingi), *Oryzias orthognathus* (buntingi), and *Oryzias profundicola.* Fish in the *Danio* genus belong to the *Cyprinidae* family and include, for example, *Danio rerio* (zebrafish), *Danio albolineatus, Danio abolineatus, Danio choprae, Danio dangila, Danio erythromicron, Danio feegradei, Danio kerri, Danio kyathit, Danio margaritatus, Danio meghalayensis, Danio nigrofasciatus,* and *Danio roseus.* Fish in the *Pimephales* genus belong to the Cyprinidae family and include *Pimephales notatus* (bluntnose minnow), *Pimephales promelas* (fathead minnow), *Pimephales tenellus* (slim minnow), and *Pimephales vigilax* (bullhead minnow). In particular embodiments, the fish embryos are Japanese or brackish medaka fish, zebrafish or fathead minnow embryos. Particular advantages of brackish medaka fish and zebrafish are described in Sections 0 and 0, respectively.

The toxicity effect can be an acute toxicityt effect (as described in Section 0) or a specific toxicity effect (as described in Section 0).

The fish embryos can be transgenic or non-transgenic. Non-transgenic fish can be used, for example, for detection of an acute toxicity effect in samples of cosmetic products or extracts from cosmetic products, e.g., toxicity, as described in Section 0. Transgenic fish embryos are particularly useful when screening for a specific effect, e.g., for detection of estrogenic compounds and anti-estrogenic compounds in samples of cosmetic products or extracts from cosmetic products as described in Section 0 below.

The screening assays can be performed in a high throughput or semi high throughput manner, *e.g.,* in multiwell plates (e.g., 24, 96 or 384 well plates), and/or with positive and/or negative controls (*e.g.,* medium only as a negative control and an agent known to exert a toxicity effect in the particular assay as a positive control). Each sample or extract in an assay can be tested in duplicate or triplicate. The assays can be performed using multiple dilutions of each sample or each extract.

### 4.5.1. Medaka fish

The brackish medaka fish (*Oryzias melastigma*) is native to coastal waters and fresh waters in Pakistan, India, Burma and Thailand (Naruse, 1996, Fish Biol. L. Medaka 8:1-9), and thrives in waters of varying salinity ranging from 0 parts per thousand (ppt) to as high as 35 ppt. Additionally, this brackish medaka fish has a number advantages for transgenic development, including: (1) small size (2-3 cm for adult fish); (2) relatively short generation time (2-3 months); (3) dimorphic sex (*e.g.,* females have a flat distal surface of the anal fin, while that of males is convex due to separated longer fin rays); (4) high prolific capacity to reproduce; (5) translucent eggs and larvae (up to 15 days post fertilization), which facilitates the positioning of DNA microinjection needles and observation of internal organs; and (6) adaptable to various transgenic techniques used to produce transgenic fish of other *Oryzias* species (*e.g., Oryzias latipes*).

Regarding the highly prolific capacity of the brackish medaka fish to reproduce, spawning of this fish can be induced all year round, and each pair of female and male fish can produce 20-30 eggs daily for up to several months under indoor maintained conditions (*e.g.,* 28±1°C with a constant light cycle of 14 h-light/8 h-dark and fed with commercial hormone-free flake food and brine shrimp (*Artemia salina*))*.* Eggs usually hatch in 11 to 15 days at 28±1°C.

The two medaka species of *Oryzias melastigma* and *Oryzias latipes* share high morphological, physiology, and genomic similarity, and while *Oryzias latipes* was first used to produce transgenic fish, the transgenic techniques were readily adapted to the brackish medaka *Oryzias melastigma* (Chen et al., 2008, Ectoxicol. Environ. Saf 71:200-208; Chen et al., 2009, Comp. Biochem. Physiol. C. Toxicol. Pharmacol. 149:647-655).

Medaka can be bred to be see-through (see, *e.g.,* U.S. Patent No. 6,737,559), further facilitating screening assays, particularly those involving detecting reporter expression or activity levels.

### 4.5.2. Zebrafish

Research has shown that zebrafish are a good model to predict toxicity of human drugs. There are close physiological and genetic similarities between zebrafish and mammalian species, and researchers have conducted systematic evaluations of zebrafish toxicity end points using large numbers of pharmacologically relevant compounds.

As an experimental tool, zebrafish have an array of advantages such as optical transparency, high fecundity, and quick, external development. Changes to morphology and modulations in gene and protein expression can be easily assayed through the use of fluorescent proteins. The relatively small physical size allows for multiple zebrafish to fit into a multiwell plate, making the scaling of experiments an easy transition. Also, the relatively cheaper costs associated with fish husbandry, coupled with the frequency of progeny that zebrafish can achieve, are other reasons that make this organism an attractive tool for screening assays.

### 4.5.3. Acute toxicity effect

Samples of cosmetic products and the cosmetic product extracts of the disclosure can be measured for acute toxic effects such as mortality and malformation on a whole organism level.

Taking zebrafish as an example, the zebrafish embryo toxicity test is based on a 48 h exposure of newly fertilized eggs in a static or semi-static system. Various endpoints such as coagulation of eggs and embryos, failure to develop somites, lack of heart-beat as well as non-detachment of the tail from the yolk are indicative of toxicity. These endpoints can be recorded after, *e.g.,* 24, 48, 72 and 96 hr and used for the calculation of an LC₅₀ value of a sample of a cosmetic product or cosmetic product extract. Analogous endpoints can be measured in Japanese medaka fish and in fathead minnows (see Braunbeck & Lammer, 2006, Background Paper on Fish Embryo Toxicity Assays, available from www.oecd.org/chemicalsafety/testing/36817242.pdf).

### 4.5.4. Specific toxicity effect

Samples of cosmetic products and the cosmetic product extracts of the disclosure can also be assayed for specific effects, *i.e.,* effects on particular tissue, organ, or hormone system. Assays of particular interest include those for cardiotoxicity, ototoxicity, seizure liability, endocrine disruption, gastrointestinal motility, hepatotoxicity, skin pigmentation alterations, muscle toxicity, pancreatic toxicity, carcinogenesis, neurotoxicity, and renal toxicity (see, *e.g.,* Sarvaiya et al., 2014, Veterinary Clinical Science 2(3):31-38, Peterson and MacRae, 2011, Annu. Rev. Pharmacol. Toxicol. 52:433-53, Eimon and Rubenstein, 2009, Expert Opin. Drug Metab. Toxicol. 5(4):393-401, and references cited therein for assay details).

In certain aspects, specific toxicity effect can be measured by detecting alterations in gene expression a result of exposure of a teleost embryo to a cosmetic product sample or cosmetic product extract. To facilitate observation of alterations in gene expression, a transgenic teleost embryo in which a regulatory sequence of interest (*e.g.,* an inducible promoter) is operably linked to a reporter sequence can be used. The regulatory sequence can be from the fish species under study or a different fish species, as long as it behaves appropriately in the fish species being assayed. Alterations in expression of the reporter following exposure of a cosmetic product sample or cosmetic product extract as compared to a (negative and/or positive) control can be detected and/or measured.

Suitable reporter sequences will be evident to those of skill in the art. For example, a suitable reporter protein can include fluorescent proteins and enzymes detectable by a histochemical method. The reporter sequences can be introduced into teleost genomes in constructs containing appropriate exogenous regulatory elements (*e.g.,* promoter and 3' untranslated regions, for example as described in U.S. Patent No. 9,043,995) or can be knocked into an endogenous genetic locus (for example using the methodology described in Kimura et al., 2014, Scientific Reports 4:6545, doi:10.1038).

Fluorescent proteins are well known in the art. Examples of fluorescent proteins include, without limitation, a green fluorescent protein (GFP), an enhanced green fluorescent protein (EGFP), a red fluorescent protein (CFP and Red FP, RFP), a blue fluorescent protein (BFP), a yellow fluorescent protein (YFP), and fluorescent variants of these proteins. The heterologous fluorescent gene (the term gene in this context refers to any coding sequence, with or without control sequences) may be, for example, a gene encoding DsRed2, ZsGreen1, and ZsYellow1. The heterologous fluorescent gene may encode any naturally occurring or variant marker proteins, including green fluorescent protein (GFP), enhanced green fluorescent protein (eGFP), yellow fluorescent protein (YFP), enhanced yellow fluorescent protein (eYFP), blue fluorescent protein (BFP), enhanced blue fluorescent protein (eBFP), cyan fluorescent protein (CFP), and enhanced cyan fluorescent protein (eCFP).

Enzymes that are detectable by histochemical methods are also well known in the art. Examples of enzymes include, without limitation, luciferase, horseradish peroxidase, β-galactosidase, β-glucuronidase, alkaline phosphatase, chloramphenicol acetyl transferase, and alcohol dehydrogenase. According to a particular embodiment, the enzyme is luciferase. The term "luciferase" is intended to denote all the proteins which catalyze or initiate a bioluminescent reaction in the presence of a substrate called luciferin. The luciferase may be from any organism or system that generates bioluminescence (see, *e.g.,* U.S. Patent No. 6,152,358). For example, the luciferase may be from *Renilla* (U.S. Patent Nos. 5,418,155 and 5,292,658), from *Photinus pyralis* or from *Luciola cruciata* (U.S. Patent No. 4,968,613).

Techniques to detect protein reporters, either directly (*e.g.,* by measuring the amount of reporter mRNA) or indirectly (*e.g.,* by measuring the amount and/or activity of the reporter protein) are conventional. Many of these methodologies and analytical techniques can be found in such references as Current Protocols in Molecular Biology, F. M. Ausubel et al., eds., (a joint venture between Greene Publishing Associates, Inc. and John Wiley & Sons, Inc.), Enzyme Immunoassay, Maggio, ed. (CRC Press, Boca Raton, 1980); Laboratory Techniques in Biochemistry and Molecular Biology, T. S. Work and E. Work, eds. (Elsevier Science Publishers B. V., Amsterdam, 1985); Principles and Practice of Immunoassays, Price and Newman, eds. (Stockton Press, NY, 1991); and the like.

In a particular embodiment, the amount and/or activity of a reporter expression product (*e.g.,* a protein) is measured. A fluorescent marker, such as eGFP, can be detected by detecting its fluorescence in the cell (*e.g.,* in a brackish medaka fish or zebrafish embryo). For example, fluorescence can be observed under a fluorescence microscope and, if desired, can be quantitated. Reporters such as eGFP, which are directly detectable without requiring the addition of exogenous factors, are preferred for detecting or assessing gene expression during fish embryonic development. A transgenic fish embryo engineered to express fluorescent reporter under the control of a promoter of interest can provide a rapid real time *in vivo* system for analyzing spatial and temporal expression patterns.

### 4.5.4.1. Endocrine Disruptor Assays

Endocrine disruptors are chemicals that, at certain doses, can interfere with the endocrine (or hormone) system in mammals. These disruptions can cause cancerous tumors, birth defects, and other developmental disorders. Specifically, endocrine disruptors may be associated with the development of learning disabilities, severe attention deficit disorder, cognitive and brain development problems; deformations of the body; breast cancer, prostate cancer, thyroid and other cancers (see Gore et al., 2015, Endocrine Reviews 36(6):593-602. doi: 10.1210/er.2015-1093). One well known example of an endocrine disruptor is bisphenol A, a chemical commonly found in plastic bottles, plastic food containers, dental materials, and the linings of metal food and infant formula cans. Bisphenol A is associated with elevated rates of diabetes, mammary and prostate cancers, decreased sperm count, reproductive problems, early puberty, obesity, and neurological problems.

Endocrine disruptors can be evaluated in transgenic teleost embryos harboring a coding sequence for a marker protein operably linked to a promoter that is sensitive to disruptors of multiple endocrine systems. Because several hormones that operate in different endocrine system share common subunits, the use of a promoter from one of the common subunits permits interrogation of multiple hormone systems simultaneously. One example of such a subunit is the glycoprotein subunit α (*gsuα*)*,* which encodes the shared α subunit of follicle stimulating hormone β, luteinizing hormone β, and thyroid-stimulating hormone (TSH) β. The *gsuα* promoter of zebrafish is an example of a promoter that can be operably linked to a coding sequence of a marker protein and used to detect endocrine disrupting chemicals (Cheng et al., 2014, Toxicology and Applied Pharmacology 278:78-84), and can be used to screen for the presence of endocrine disrupting chemicals in cosmetic products as described herein.

Many endocrine disruptors possess estrogenic, enhancing-estrogenic or anti-estrogenic properties. For the evaluation of the estrogenic, enhancing-estrogenic and anti-estrogenic properties of cosmetic product samples and cosmetic product extracts of the disclosure, the cosmetic product samples or cosmetic product extracts can be assayed in teleost embryos harboring an estrogen responsive promoter operably linked to a coding sequence for a marker protein. In some embodiments, the estrogen responsive promoter is from a choriogenin gene of a medaka fish (*e.g., Oryzias melastigma* and *Oryzias latipes*), for example choriogenin H or choriogenin L. Choriogenin H and L are precursor proteins of the inner layer subunits of egg envelope (chorion) of teleost fish, and gene expression of both choriogenin H and choriogenin L are responsive to estrogenic substances (see, *e.g.,* Yamaguchi et al., 2015, J Appl Toxicol. 35(7):752-8). In some embodiments, the choriogenin H promoter is used to assay the estrogen disruptor activity of a cosmetic product sample or cosmetic product extract. The choriogenin H promoter has been shown to be a highly sensitive biomarker for monitoring estrogenic chemicals in the marine environment (Chen et al., 2008, Ecotoxicol Environ Saf. 71(1):200-8). Examples of choriogenin H promoter constructs suitable for use for assaying estrogenic activity of cosmetic product samples and cosmetic product extracts are disclosed in U.S. Patent No. 9,043,995. In other embodiments, the choriogenin L promoter is used. In other embodiments, the estrogen responsive promoter is the brain aromatase B promoter (referred to as a cyp19a1b promoter in zebrafish). The zebrafish cyp19a1b gene exhibits exquisite sensitivity to estrogens and is a sensitive target for estrogen mimics, and has been successfully operably linked to a marker gene such as GFP in transgenic fish (see, *e.g.,* Brion et al., 2012, PLoS ONE 7(5): e36069. doi:10.1371/journal.pone.0036069). In yet another embodiment, the estrogen sensitive promoter is a vitellogenin promoter (for example as described in Schreurs et al., 2004, Environmen. Sci. Technol. 34:4439-44).

Other endocrine disruptors possess androgenic, enhancing-androgenic or anti-androgenic properties. Androgenic, enhancing-androgenic and anti-androgenic properties of cosmetic product samples and cosmetic product extracts can be evaluated in teleost embryos harboring an androgen responsive promoter operably linked to a coding sequence for a marker protein. In some embodiments, the androgen responsive promoter is the *G. aculeatus spiggin* promoter, which is responsive to androgens but exhibits no reactivity to, *inter alia,* estrogens and glucocorticoids (*see, e.g.,* Sebillot et al., 2014, Environ. Sci. Technol. 48:10919-28).

Yet other endocrine disruptors possess thyroid-disrupting properties, *e.g*., they disrupt the hypothalamic-pituitary-thyroid (HPT) axis. Thyroid/HPT disrupting properties of cosmetic product samples and cosmetic product extracts can be evaluated in teleost embryos harboring a thyroid hormone (TH) responsive promoter operably linked to a coding sequence for a marker protein. In some embodiments, the thyroid responsive promoter is the thyroid-stimulating hormone subunit β (TSHβ) promoter, which in contrast to other subunits is unique to TSH. Thyroid-stimulating hormone is part of a feedback loop involving TH and thyrotropin-releasing hormone (TRH). Specifically, when low levels of TH are present, TRH is secreted by the hypothalamus to stimulate the release of TSH by the pituitary, which in turn stimulates the thyroid to secrete TH, and the opposite feedback loop occurs when high levels of TH are present. The TSHβ promoter is a useful biomarker for the HPT axis. An example of a TSH β promoter that can be used is the zebrafish TSH β promoter (*see, e.g.,* Ji et al., 2012, Toxicology and Applied Pharmacology 262:149-155.

### 4.5.4.2. Xenobiotic Assays

Xenobiotics are foreign chemical substances present within an organism. Xenobiotics may be grouped as antioxidants, carcinogens, drugs, environmental pollutants, food additives, hydrocarbons, and pesticides. Pollutants such as dioxins and polychlorinated biphenyls are considered xenobiotics. The body removes xenobiotics by xenobiotic metabolism. This consists of the deactivation and the excretion of xenobiotics, and happens mostly in the liver, by way of reactions catalyzed by the hepatic microsomal cytochrome P450 enzyme system.

For the evaluation of the xenobiotic properties of cosmetic product samples and cosmetic product extracts of the disclosure, the cosmetic product samples and cosmetic extracts can be assayed in teleost embryos harboring a xenobiotic responsive promoter operably linked to a coding sequence for a marker protein. In some embodiments, the promoter is a cytochrome P450 promoter, *e.g.,* the zebrafish P450 1A (Cypla) promoter such as described in Boon and Gong, 2013, PLOS ONE 8(5):e64334.

Xenobiotic properties of cosmetic product samples and cosmetic product extracts of the disclosure can also be evaluated using an *in vivo* ethoxyresorufin-O-deethylase (EROD) activity assay using 7-ethoxyresorufin as substrate, for example as described in Liu et al., 2014, Environmental Toxicology 31(2):201-10.

### 4.5.4.3. Hepatotoxicity assays

Zebrafish have been studies as models of drug-induced hepatotoxicity. The transparency of zebrafish for several days post-fertilization enables *in* vivo visual observation of internal organs including liver. Zebrafish complete primary liver morphogenesis by 48 hours post-fertilization (HPF). When exposed to a hepatotoxicant, changes to liver morphology can be evaluated visually (Hill et al., 2012, Drug Metabolism Reviews 44(1):127-140). Researchers have developed various endpoints that can be studied to evaluate hepatotoxicity: liver degeneration, changes in size and shape of the liver, and yolk sac retention (see He et al., 2013, Journal of Pharmacological and Toxicological Methods 67:25-32). These parameters can be assayed in zebrafish to evaluate the hepatotoxicity of a cosmetic product sample or cosmetic product extract of the disclosure, and analogous parameters can be used to assay the hepatotoxicity of a cosmetic product sample or cosmetic product extract in a different fish such as medaka.

### 4.5.4.4. Cardiotoxicity assays

Teleost embryos provide an ideal model system for investigating cardiotoxicity because their transparency and uncovered hearts make them easily observable. Taking zebrafish as an example, the heart consists of a ventricle and an atrium and these develop rapidly. Heart tube and heartbeat are observed at 24 hours post fertilization (hpf), and then tube looping, chamber formation, and blood circulation are completed by 72 hpf.

It is possible to assay cosmetic product samples and cosmetic product extracts for cardiotoxicity by evaluating parameters such as heart rate, rhythmicity (*e.g.,* atrioventricular block (AV block), arrhythmia); circulation, and morphology (*e.g.,* pericardial edema; hemorrhage, heart chamber swelling) in teleost embryos.

The heart-specific promoter BMP4 can be used to drive expression of a marker gene that allows heart morphology to be observed. The erythrocyte-specific promoter gata1 can be used to drive expression of a marker gene, allowing the blood circulation rate to be observed (see Wu et al., 2013, Toxicol. Sci. 136(2):402-412, and references cited therein).

These parameters can be assayed in zebrafish or medaka to evaluate the cardiotoxicity of a cosmetic product sample or a cosmetic product extract of the disclosure.

### EXAMPLES

### 5.1. Example 1: BB cream extract preparation

Three BB creams of SPF50 produced by brands A, B and C were extracted and tested for acute toxicity and estrogenic activity. BB creams were extracted with 3 times volume of methanol. Each sample was vortexed, sonicated and centrifuged. The supernatants were collected and stored at -20°C until testing.

### 5.2. Example 2: Acute toxicity testing of BB cream extracts

Extracts prepared as described in Example 1 were tested for acute toxicity using zebrafish (*Danio rerio*) AB strain embryos. BB cream extracts were diluted into zebrafish embryo culture medium at nominal concentrations of 0.3, 0.6, 1.4, 3.0 and 6.7 g/L. Zebrafish AB strain embryos of 4-128 cell stages were exposed to extract dilutions in a 96-well plate at 1 embryo per well. Each concentration was tested with 20 embryos. Zebrafish embryo culture medium and 3.7 mg/L dichloroaniline were included as negative and positive controls, respectively. After 48 hr exposure at 26°C, zebrafish embryos were observed under a stereomicroscope and fish embryos that were coagulated, tail not detached, and having no heart beat were marked as dead. Mortality rate for each concentration was calculated as the acute toxicity endpoint. The mortality rate for the negative control was 0% and for the positive control was 30%. Table 1 shows the acute toxicity test results. Of the three BB cream extracts, brand C was the most toxic and brand B was the least toxic to zebrafish embryos.

| **Table 1: Acute toxicity of BB cream extracts from brands A, B and C** | | | | | | |
|---|---|---|---|---|---|---|
| Tested extract concentration | | 0.3 g/L | 0.6 g/L | 1.4 g/L | 3.0 g/L | 6.7 g/L |
| Mortality rate of each Sample | Brand A | 0% | 5% | 5% | 95% | 100% |
| | Brand B | 0% | 0% | 0% | 5% | 20% |
| | Brand C | 0% | 5% | 10% | 100% | 100% |

### 5.3. Example 3: Estrogenic activity of BB cream extract

Extracts prepared as described in Example 1 were tested for estrogenic activity using choriogenin H - eGFP transgenic medaka (*Oryzias melastigma*) eleutheroembryos generated as described in Example 1 of U.S. Patent No. 9,043,995. BB cream extracts were diluted into medaka (*Oryzias melastigma*) embryo culture medium at nominal concentration of 0.33 g/L. 17β-estradiol was also tested at 1.0, 2.0, 5.0 and 10.0 µg/L as positive controls. Culture medium was tested as negative control. Each concentration contained 3 replicates with each replicate containing 8 eleutheroembryos. After 24-hr exposure at 26°C, eleutheroembryos were observed under green fluorescence microscope and imaged from ventral side using the same imaging setting. Negative control and extracts of samples from brand B did not induce observable green fluorescence in the eleutheroembryo livers. The extracts of the samples from brand A and brand C induced observable green fluorescence in the eleutheroembryo livers.

### 5.4. Example 4: Assay for sunscreen

Brands A, B and C of sunscreen of SPF50 were extracted for acute toxicity and estrogenic activity testing. Suncreen was mixed with 1:3 (w/v) methanol. After vortexing and sonication, samples were centrifuged to separate the phases. The supernatant was separated and stored at -20°C until testing.

The acute toxicity and estrogenic activity testing of the sunscreen samples were conducted according to the methods described in the above sections 5.2 (Example 2) and 5.3 (Example 3) and the results show that it can be identified whether a toxicant is present in the sunscreen. The acute toxicity and estrogenic activity data of sunscreens are shown in Table 2 and Table 3 below.

| **Table 2: Acute toxicity of 3 sunscreens** | | | | | | |
|---|---|---|---|---|---|---|
| Tested sample nominal concentration | | 0.3 g/L | 0.6 g/L | 1.4 g/L | 3.0 g/L | 6.7 g/L |
| Mortality rate of each sample | Brand A | 0% | 0% | 5% | 0% | 80% |
| | Brand B | 5% | 0% | 0% | 10% | 30% |
| | Brand C | 0% | 10% | 30% | 60% | 100% |

| **Table 3: Estrogen equivalent concentration of 3 sunscreens** | | |
|---|---|---|
| Estrogen equivalent concentration of each sample | Brand A | Not detected |
| | Brand B | 1,400 ng/g |
| | Brand C | 170,000 ng/g |

### 5.5 Example 5: Assay for paper masks

Brands A, B and C of paper masks were squeezed to get solution for acute toxicity and estrogenic activity testing; or mixed with methanol at 1:3 (w/v), vortexed, sonicated and centrifuged to get supernatant for testing.

The acute toxicity and estrogenic activity testing of the paper mask samples were conducted according to the methods described in the above sections 5.2 (Example 2) and 5.3 (Example 3) and the results show that it can be identified whether a toxicant is present in the paper masks. The acute toxicity and estrogenic activity data of paper masks are shown in Table 4 and Table 5 below.

| **Table 4: Acute toxicity of 3 paper masks** | | | | | | |
|---|---|---|---|---|---|---|
| Tested sample nominal concentration | | 0.3 g/L | 0.6 g/L | 1.4 g/L | 3.0 g/L | 6.7 g/L |
| Tested sample nominal concentration | | 0.3 g/L | 0.6 g/L | 1.4 g/L | 3.0 g/L | 6.7 g/L |
| Mortality rate of each sample | Brand A | 50% | 100% | 100% | 100% | 100% |
| | Brand B | 0% | 10% | 30% | 60% | 80% |
| | Brand C | 10% | 10% | 10% | 10% | 10% |

| **Table 5: Estrogen equivalent concentration of 3 paper masks** | | |
|---|---|---|
| Estrogen equivalent concentration of each sample | Brand A | Not detected |
| | Brand B | 1,400 ng/g |
| | Brand C | 170,000 ng/g |

### 5.6 Example 6: Assay for baby cream

Brands A, B and C of baby cream were extracted for acute toxicity and estrogenic activity testing. Baby cream was mixed with 1:3 (w/v) methanol. After vortexing and sonication, samples were centrifuged to separate the phases. The supernatant was separated and stored at -20°C until testing.

The acute toxicity and estrogenic activity testing of the baby cream samples were conducted according to the methods described in the above sections 5.2 (Example 2) and 5.3 (Example 3) and the results show that it can be identified whether a toxicant is present in the baby cream. The acute toxicity data and estrogenic activity of baby cream are shown in Table 6 and Table 7 below.

| **Table 6: Acute toxicity of 3 baby cream** | | | | | | |
|---|---|---|---|---|---|---|
| Tested sample nominal concentration | | 0.3 g/L | 0.6 g/L | 1.4 g/L | 3.0 g/L | 6.7 g/L |
| Mortality rate of each sample | Brand A | 0% | 0% | 40% | 70% | 100% |
| | Brand B | 0% | 10% | 0% | 10% | 40% |
| | Brand C | 0% | 0% | 10% | 50% | 70% |

| **Table 7: Estrogen equivalent concentration of 3 baby cream** | | |
|---|---|---|
| Estrogen equivalent concentration of each sample | Brand A | Not detected |
| | Brand B | 1,500 ng/g |
| | Brand C | Not detected |

### 5.7 Example 7: Assay for baby oil

Brands A, B and C of baby oil were extracted for acute toxicity and estrogenic activity testing. Baby oil was mixed with 1:3 (w/v) methanol. After vortexing and sonication, samples were centrifuged to separate the phases. The supernatant was separated and stored at -20°C until testing.

The acute toxicity and estrogenic activity testing of the baby oil samples were conducted according to the methods described in the above sections 5.2 (Example 2) and 5.3 (Example 3) and the results show that it can be identified whether a toxicant is present in the baby oils. The acute toxicity and estrogenic activity data of baby oil are shown in Table 8 and Table 9 below.

| **Table 8: Acute toxicity of 3 baby oils** | | | | | | |
|---|---|---|---|---|---|---|
| Tested sample nominal concentration | | 0.3 g/L | 0.6 g/L | 1.4 g/L | 3.0 g/L | 6.7 g/L |
| Mortality rate of each sample | Brand A | 0% | 0% | 10% | 10% | 30% |
| | Brand B | 0% | 5% | 0% | 0% | 10% |
| | Brand C | 0% | 0% | 10% | 35% | 60% |

| **Table 9: Estrogen equivalent concentration of 3 baby oils** | | |
|---|---|---|
| Estrogen equivalent concentration of each sample | Brand A | Not detected |
| | Brand B | Not detected |
| | Brand C | Not detected |

### 5.8 Example 8: Assay for antiperspirant

Brands A, B and C of antiperspirant were extracted by mixed with 1:3 (w/v) methanol, vortexed, sonicated, and centrifuged, use the supernatant for testing; or no sample pretreatment was performed before dilution to fish medium for testing.

The acute toxicity and estrogenic activity testing of the antiperspirant samples were conducted according to the methods described in the above sections 5.2 (Example 2) and 5.3 (Example 3) and the results show that it can be identified whether a toxicant is present in the antiperspirant. The acute toxicity data and estrogenic activity of antiperspirant are shown in Table 10 and Table 11 below.

| **Table 10: Acute toxicity of 3 antiperspirants** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 20% | 60% | 100% | 100% | 100% |
| | Brand B | 0% | 5% | 20% | 35% | 70% |
| | Brand C | 100% | 100% | 100% | 100% | 100% |

| **Table 11: Estrogen equivalent concentration of 3 antiperspirants** | | |
|---|---|---|
| Estrogen equivalent concentration of each sample | Brand A | Not detected |
| | Brand B | Not detected |
| | Brand C | Not detected |

### 5.9 Example 9: Assay for lipstick

Brands A, B and C of lipsticks were extracted for acute toxicity and estrogenic activity testing. Lipstick was mixed with 1:3 (w/v) methanol. After vortexing and sonication, samples were centrifuged to separate the phases. The supernatant was separated and stored at -20°C until testing.

The acute toxicity and estrogenic activity testing of the lipstick samples were conducted according to the methods described in the above sections 5.2 (Example 2) and 5.3 (Example 3) and the results show that it can be identified whether a toxicant is present in the lipstick. The acute toxicity data and estrogenic activity of lipstick are shown in Table 12 and Table 13 below.

| **Table 12: Acute toxicity of 3 lipsticks** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 0% | 10% | 5% | 20% | 60% |
| | Brand B | 0% | 5% | 0% | 5% | 0% |
| | Brand C | 0% | 5% | 10% | 0% | 20% |

| **Table 13: Estrogen equivalent concentration of 3 lipsticks** | | |
|---|---|---|
| Estrogen equivalent concentration of each sample | Brand A | Not detected |
| | Brand B | 1,000 ng/g |
| | Brand C | Not detected |

### 5.10 Example 10: Assay for baby ointment

Brands A, B and C of baby ointment were extracted for acute toxicity and estrogenic activity testing. Ointment was mixed with 1:3 (w/v) methanol. After vortexing and sonication, samples were centrifuged to separate the phases. The supernatant was separated and stored at -20°C until testing.

The acute toxicity and estrogenic activity testing of the ointment samples were conducted according to the methods described in the above sections 5.2 (Example 2) and 5.3 (Example 3) and the results show that it can be identified whether a toxicant is present in the ointment. The acute toxicity and estrogenic activity data of ointments are shown in Table 14 and Table 15 below.

| **Table 14: Acute toxicity of 3 ointments** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 0% | 0% | 10% | 15% | 40% |
| | Brand B | 0% | 5% | 30% | 5% | 60% |
| | Brand C | 0% | 5% | 10% | 0% | 25% |

| **Table 15: Estrogen equivalent concentration of 3 ointments** | | |
|---|---|---|
| Estrogen equivalent concentration of each sample | Brand A | Not detected |
| | Brand B | Not detected |
| | Brand C | Not detected |

### 5.11 Example 11: Assay for lotion

Brands A, B and C of lotion were extracted by mixed with 1:3 (w/v) methanol, vortexed, sonicated, and centrifuged to get supernatant for testing; or no sample pretreatment was performed before dilution to fish medium for testing.

The acute toxicity and estrogenic activity testing of the lotion samples were conducted according to the methods described in the above sections 5.2 (Example 2) and 5.3 (Example 3) and the results show that it can be identified whether a toxicant is present in the lotion. The acute toxicity and estrogenic activity data of lotions are shown in Table 16 and Table 17 below.

| **Table 16: Acute toxicity of 3 lotions** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 0% | 0% | 0% | 5% | 20% |
| | Brand B | 0% | 5% | 10% | 35% | 60% |
| | Brand C | 0% | 15% | 60% | 100% | 100% |

| **Table 17: Estrogen equivalent concentration of 3 lotions** | | |
|---|---|---|
| Estrogen equivalent concentration of each sample | Brand A | 900 ng/g |
| | Brand B | Not detected |
| | Brand C | 2,300 ng/g |

### 5.12 Example 12: Assay for make-up remover

Brands A (liquid type), B (oil type) and C (powder type) of make-up remover were extracted by mixed with 1:3 (w/v) methanol, vortexed, sonicated, and centrifuged to get supernatant for testing.

The acute toxicity and estrogenic activity testing of the make-up remover samples were conducted according to the methods described in the above sections 5.2 (Example 2) and 5.3 (Example 3) and the results show that it can be identified whether a toxicant is present in the make-up remover. The acute toxicity and estrogenic activity data of make-up removers are shown in Table 18 and Table 19 below.

| **Table 18: Acute toxicity of 3 make-up removers** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 0% | 5% | 30% | 100% | 100% |
| | Brand B | 100% | 100% | 100% | 100% | 100% |
| | Brand C | 0% | 5% | 0% | 25% | 50% |

| **Table 19: Estrogen equivalent concentration of 3 make-up removers** | | |
|---|---|---|
| Estrogen equivalent concentration of each sample | Brand A | 800 ng/g |
| | Brand B | Not detected |
| | Brand C | 2,700 ng/g |

### 5.13 Example 13: Assay for essential oils

Brands A, B and C of essential oils were extracted by mixed with 1:3 (w/v) methanol, vortexed, sonicated, and centrifuged to get supernatant for testing.

The acute toxicity and estrogenic activity testing of the essential oil samples were conducted according to the methods described in the above sections 5.2 (Example 2) and 5.3 (Example 3) and the results show that it can be identified whether a toxicant is present in the essential oil. The acute toxicity and estrogenic activity data of essential oil are shown in Table 20 and Table 21 below.

| **Table 20: Acute toxicity of 3 essential oils** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 0% | 5% | 0% | 10% | 20% |
| | Brand B | 0% | 10% | 50% | 0% | 0% |
| | Brand C | 0% | 5% | 0% | 10% | 70% |

| **Table 21: Estrogen equivalent concentration of 3 essential oils** | | |
|---|---|---|
| Estrogen equivalent concentration of each sample | Brand A | 1,800 ng/g |
| | Brand B | Not detected |

| **Table 20: Acute toxicity of 3 essential oils** | | | | | | | |
|---|---|---|---|---|---|---|---|
| Tested sample nominal concentration | 0.21 g/L | | 0.6 g/L | 1.4 g/L | | 3.0 g/L | 6.7 g/L |
| | | Brand C | | | 700 ng/g | | |

### 5.14 Example 14: Assay for essence

Brands A, B and C of essence were extracted by mixed with 1:3 (w/v) methanol, vortexed, sonicated, and centrifuged to get supernatant for testing.

The acute toxicity and estrogenic activity testing of the essence samples were conducted according to the methods described in the above sections 5.2 (Example 2) and 5.3 (Example 3) and the results show that it can be identified whether a toxicant is present in the essence. The acute toxicity and estrogenic activity data of essence are shown in Table 22 and Table 23 below.

| **Table 22: Acute toxicity of 3 essences** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 100% | 100% | 100% | 100% | 100% |
| | Brand B | 5% | 0% | 10% | 50% | 80% |
| | Brand C | 0% | 5% | 0% | 10% | 30% |

| **Table 23: Estrogen equivalent concentration of 3 essences** | | |
|---|---|---|
| Estrogen equivalent concentration of each sample | Brand A | 3,800 ng/g |
| | Brand B | Not detected |
| | Brand C | 17,000 ng/g |

### 5.15 Example 15: Assay for perfume

Brands A, B and C of perfume were extracted by mixed with 1:3 (w/v) methanol, vortexed, sonicated, and centrifuged to get supernatant for testing.

The acute toxicity and estrogenic activity testing of the perfume samples were conducted according to the methods described in the above sections 5.2 (Example 2) and 5.3 (Example 3) and the results show that it can be identified whether a toxicant is present in the perfume. The acute toxicity and estrogenic activity data of perfume are shown in Table 24 and Table 25 below.

| **Table 24: Acute toxicity of 3 perfumes** | | | | | | |
|---|---|---|---|---|---|---|
| Tested sample nominal concentration | | 0.21 g/L | 0.6 g/L | 1.4 g/L | 3.0 g/L | 6.7 g/L |
| Mortality rate of each sample | Brand A | 100% | 100% | 100% | 100% | 100% |
| | Brand B | 0% | 10% | 50% | 100% | 100% |
| | Brand C | 0% | 15% | 100% | 100% | 100% |

| **Table 25: Estrogen equivalent concentration of 3 perfumes** | | |
|---|---|---|
| Estrogen equivalent concentration of each sample | Brand A | Not detected |
| | Brand B | 1,400 ng/g |
| | Brand C | Not detected |

### 5.16 Example 16: Assay for shampoo

Brands A, B and C of shampoo were extracted for acute toxicity and estrogenic activity testing. Shampoo was mixed with 1:3 (w/v) methanol. After vortexing and sonication, samples were centrifuged to separate the phases. The supernatant was separated and stored at -20°C until testing.

The acute toxicity and estrogenic activity testing of the shampoo samples were conducted according to the methods described in the above sections 5.2 (Example 2) and 5.3 (Example 3) and the results show that it can be identified whether a toxicant is present in the shampoo. The acute toxicity and estrogenic activity data of shampoo are shown in Table 26 and Table 27 below.

| **Table 26: Acute toxicity of 3 shampoos** | | | | | | |
|---|---|---|---|---|---|---|
| Tested sample nominal concentration | | 0.03 g/L | 0.06 g/L | 0.1 g/L | 0.3 g/L | 0.7 g/L |
| Mortality rate of each sample | Brand A | 30% | 100% | 100% | 100% | 100% |
| | Brand B | 0% | 5% | 10% | 20% | 30% |
| | Brand C | 0% | 0% | 10% | 30% | 95% |

| **Table 27: Estrogen equivalent concentration of 3 shampoos** | | |
|---|---|---|
| Estrogen equivalent concentration of each sample | Brand A | Not detected |
| | Brand B | 3,000 ng/g |
| | Brand C | Not detected |

## Claims

1. A method of determining the overall toxicity in a cosmetic product, comprising:
a) combining a solvent and a sample of the cosmetic product to obtain a solvent extract with an overall toxicity;
b) contacting a teleost embryo with the solvent extract of a); and
c) determining whether the extract exerts a toxicity effect on the embryo;
wherein a toxicity effect on the embryo shows an overall toxicity of the cosmetic product;
and wherein the extract is (i) an organic solvent extract or (ii) an organic and inorganic solvent extract;
wherein the organic solvent is methanol, DMSO, acetonitrile or acetone, and wherein the inorganic solvent is water; wherein when the sample of the cosmetic product is a liquid, the volume of the solvent is about 1.5 to about 10 times the volume of the sample; or when the sample of the cosmetic product is a solid or semi-solid, the volume of the solvent is about 1.5 to about 10 times the weight of the sample.

2. The method of claim 1, wherein the cosmetic product is mask, perfume, antiperspirant, deodorant, toothpaste, shampoo, essential oil, make-up remover, cleansing product, hair dye, foundation, concealer, sunscreen, moisturizer, anti-wrinkle cream, eyeshadow, eyeliner, mascara, blush, lipstick, lip gloss, nail polish, serum, eye cream, night cream, day cream, BB cream, night lotion, day lotion, hand cream, neck cream, or anti-aging cream.

3. The method of claim 1 or claim 2, wherein the cosmetic product is (i) a water miscible cosmetic product or (ii) not a water miscible cosmetic product.

4. The method of any one of claims 1 to 3, wherein the extract is obtainable by a process comprising (a) combining a sample of the cosmetic product with the organic solvent to form a mixture, (b) mixing the mixture, optionally by vortexing and/or sonicating the mixture, (c) separating a phase containing the organic solvent from the mixture, optionally by centrifuging the mixture, and (d) recovering the extract, optionally wherein the extract is the supernatant or a concentrated form thereof.

5. The method of any one of claims 1 to 4, wherein the organic and inorganic solvent are used in a ratio of 3:7 to 7:3, optionally wherein the ratio is 4:6 to 6:4.

6. The method of any one of claims 1 to 5, further comprising, prior to step (a), preparing the extract from the sample of the cosmetic product.

7. The method of any one of claims 1 to 6, wherein the cosmetic is a water miscible liquid cosmetic product and wherein step (a) comprises contacting a teleost embryo with a sample of the cosmetic product.

8. The method of any one of claims 1 to 7, wherein the teleost embryo is:
a) an eleutheroembryo; and/or
b) a medaka embryo, optionally which is a transgenic medaka embryo; a zebrafish embryo, optionally which is a transgenic zebrafish embryo; or a fathead minnow embryo.

9. The method of any one of claims 1 to 8, wherein the toxicity effect comprises an acute effect, which optionally comprises mortality, malformation or a combination thereof; or a specific effect.

10. The method of claim 9, wherein the specific effect is an endocrine activity disruption, which is optionally estrogen activity disruption, androgen activity disruption or thyroid activity disruption, optionally wherein the teleost embryo is a transgenic teleost embryo comprising a glycoprotein subunit α (*gsuα*) promoter operably linked to a marker gene.

11. The method of claim 9, wherein the specific effect is an estrogen activity disruption, wherein the teleost embryo is a transgenic teleost embryo comprising an estrogen sensitive promoter operably linked to a marker gene.

12. The method of claim 11, wherein the estrogen sensitive promoter is:
a) an aromatase B promoter, and optionally wherein the teleost embryo is a zebrafish embryo or a medaka embryo;
b) a choriogenin promotor, which is optionally a choriogenin H promoter or a choriogenin L promoter, and optionally wherein the teleost embryo is a medaka embryo; or
c) a vitellogenin promoter, and optionally wherein the teleost embryo is a zebrafish embryo or a medaka embryo.

13. The method of claim 9, wherein the specific effect is androgen activity disruption, wherein the teleost embryo is a transgenic teleost embryo comprising an androgen sensitive promoter operably linked to a marker gene, optionally wherein the androgen sensitive promoter is a *spiggin* promoter and optionally wherein the teleost embryo is a medaka embryo or a zebrafish embryo.

14. The method of claim 9, wherein the specific effect is thyroid activity disruption, wherein the teleost embryo is a transgenic teleost embryo comprising a thyroid hormone (TH) sensitive promoter operably linked to a marker gene, optionally wherein the TH sensitive promoter is a thyroid-stimulating hormone subunit β (TSHβ) promoter, and optionally wherein the teleost embryo is a medaka embryo or a zebrafish embryo.

15. The method of claim 9, wherein the specific effect is a xenobiotic effect, wherein the teleost embryo is a transgenic teleost embryo comprising a xenobiotic sensitive promoter operably linked to a marker gene, optionally wherein the xenobiotic sensitive promoter is a P450 1A promoter, and optionally wherein the teleost embryo is a medaka embryo or a zebrafish embryo.

16. The method of any one of claims 1 to 15, wherein determining whether the sample or the extract exerts a toxicity effect on the embryo comprises detecting or measuring changes in expression of the marker gene.

17. The method of claim 9, wherein the specific effect is a xenobiotic effect, wherein determining whether the sample or the extract exerts a toxicity effect on the embryo comprises detecting or measuring changes in ethoxyresorufin-O-deethylase (EROD) activity, and optionally wherein the teleost embryo is a medaka embryo or a zebrafish embryo.

18. The method of claim 9, wherein the specific effect is a cardiotoxicity effect, optionally wherein determining whether the sample or the extract exerts a toxicity effect on the embryo comprises detecting or measuring alterations in cardiac development and/or blood circulation rate.

19. The method of claim 18, wherein:
a) the embryo harbors a BMP4 promoter operably linked to a marker gene and wherein detecting or measuring alterations in cardiac development comprises monitoring marker gene expression; or
b) the embryo harbors a gata1 promoter operably linked to a marker gene and wherein detecting or measuring alterations in blood circulation rate comprises monitoring marker gene expression.

20. The method of claim 9, wherein the specific effect is a hepatotoxicity effect, and optionally wherein determining whether the sample or the extract exerts a toxicity effect on the embryo comprises detecting or measuring changes in liver development.

21. The method of any one of claims 10-16 and 19, wherein the marker gene encodes:
a) a fluorescent protein, which is optionally a green fluorescent protein (GFP), cyan fluorescent protein (CFP), yellow fluorescent protein (YFP), red fluorescent protein (dsRFP), luciferase (Luc), chloramphenicol acetyltransferase (CAT), 13-galactosidase (LacZ) or β-glucuronidase (Gus); or
b) an enzyme detectable in a colorimetric assay, optionally wherein the enzyme is a luciferase, horseradish peroxidase, β-galactosidase, β-glucuronidase, alkaline phosphatase, chloramphenicol acetyl transferase, or alcohol dehydrogenase.

22. The method of any one of claims 10-16 and 19, wherein the promoter is native to the teleost embryo.

23. The method of any one of claims 10-16 and 19, wherein the promoter is not native to the teleost embryo, optionally wherein:
a) the teleost embryo is a zebrafish embryo and the promoter is native to a medaka fish, wherein the medaka fish is optionally *Oryzias melastigma* or *Oryzias latipes;* or
b) the teleost embryo is a medaka embryo and the promoter is native to a zebrafish, wherein the medaka embryo is optionally an *Oryzias melastigma* embryo or *Oryzias latipes* embryo.

24. The method of any one of claims 1 to 23, wherein the method is performed in a multiwell plate, optionally a 24-well plate, 96-well plate or a 384-well plate.

25. The method of any one of claims 1 to 24, wherein more than one cosmetic product sample is assayed, optionally in which each sample is assayed in duplicate or in triplicate.

26. The method of any one of claims 1 to 24, wherein the method comprises assaying multiple dilutions of a cosmetic product sample or extract.

27. The method of any one of claims 1 to 21, which comprises any combination of the methods of claims 24-26.

## Patentansprüche

1. Verfahren zur Bestimmung der Gesamttoxizität in einem kosmetischen Produkt, umfassend:
a) Kombination eines Lösungsmittels und einer Probe des kosmetischen Produkts, um einen Lösungsmittelextrakt mit einer Gesamttoxizität zu erhalten;
b) Kontaktieren eines Teleostenembryos mit dem Lösungsmittelextrakt von a); und
c) Bestimmen, ob der Extrakt eine toxische Wirkung auf den Embryo ausübt;
wobei eine Toxizitätswirkung auf den Embryo eine Gesamttoxizität des kosmetischen Produkts zeigt; und wobei der Extrakt (i) ein organischer Lösungsmittelextrakt oder (ii) ein organischer und anorganischer Lösungsmittelextrakt ist;
wobei das organische Lösungsmittel Methanol, DMSO, Acetonitril oder Aceton ist und wobei das anorganische Lösungsmittel Wasser ist; wobei, wenn die Probe des kosmetischen Produkts eine Flüssigkeit ist, das Volumen des Lösungsmittels etwa das 1,5- bis etwa das 10-fache des Volumens der Probe beträgt; oder, wenn die Probe des kosmetischen Produkts ein Feststoff oder Halbfeststoff ist, das Volumen des Lösungsmittels etwa das 1,5- bis etwa das 10-fache des Gewichts der Probe beträgt.

2. Verfahren nach Anspruch 1, wobei das kosmetische Produkt eine Maske, ein Parfüm, ein Antitranspirant, ein Deodorant, eine Zahnpasta, ein Shampoo, ein ätherisches Öl, ein Make-up-Entferner, ein Reinigungsprodukt, ein Haarfärbemittel, eine Grundierung, ein Abdeckstift, ein Sonnenschutzmittel, eine Feuchtigkeitscreme, eine Antifaltencreme, ein Lidschatten, ein Eyeliner, ein Mascara, ein Rouge, ein Lippenstift, ein Lipgloss, ein Nagellack, ein Serum, eine Augencreme, eine Nachtcreme, eine Tagescreme, eine BB-Creme, eine Nachtlotion, eine Tageslotion, eine Handcreme, eine Halscreme oder eine Anti-Aging-Creme ist.

3. Verfahren nach Anspruch 1 oder Anspruch 2, wobei das kosmetische Produkt (i) ein wassermischbares kosmetisches Produkt oder (ii) kein wassermischbares kosmetisches Produkt ist.

4. Verfahren nach einem der Ansprüche 1 bis 3, wobei der Extrakt durch ein Verfahren erhältlich ist, das Folgendes umfasst: (a) Kombinieren einer Probe des kosmetischen Produkts mit dem organischen Lösungsmittel, um eine Mischung zu bilden, (b) Mischen der Mischung, optional durch Verwirbeln und/oder Beschallen der Mischung, (c) Abtrennen einer Phase, die das organische Lösungsmittel enthält, von der Mischung, optional durch Zentrifugieren der Mischung, und (d) Gewinnen des Extrakts, wobei der Extrakt optional der Überstand oder eine konzentrierte Form davon ist.

5. Verfahren nach einem der Ansprüche 1 bis 4, wobei das organische und anorganische Lösungsmittel in einem Verhältnis von 3:7 bis 7:3 verwendet werden, wobei das Verhältnis optional 4:6 bis 6:4 beträgt.

6. Verfahren nach einem der Ansprüche 1 bis 5, ferner umfassend (vor dem Schritt (a)) die Herstellung des Extrakts von der Probe des kosmetischen Produkts.

7. Verfahren nach einem der Ansprüche 1 bis 6, wobei das Kosmetikum ein wassermischbares flüssiges kosmetisches Produkt ist und wobei Schritt (a) das Kontaktieren eines Teleostenembryos mit einer Probe des kosmetischen Produkts umfasst.

8. Verfahren nach einem der Ansprüche 1 bis 7, wobei der Teleostenembryo Folgendes ist:
a) ein Eleutheroembryo; und/oder
b) ein Medakaembryo, der optional ein transgener Medakaembryo ist; ein Zebrafischembryo, der optional ein transgener Zebrafischembryo ist; oder ein Dickkopfelritzenembryo.

9. Verfahren nach einem der Ansprüche 1 bis 8, wobei die Toxizitätswirkung eine akute Wirkung, die optional Mortalität, Fehlbildung oder eine Kombination davon umfasst, oder eine spezifische Wirkung umfasst.

10. Verfahren nach Anspruch 9, wobei die spezifische Wirkung eine Störung der endokrinen Aktivität ist, die optional eine Störung der Östrogenaktivität, eine Störung der Androgenaktivität oder eine Störung der Schilddrüsenaktivität ist, wobei der Teleostenembryo optional ein transgener Teleostenembryo ist, der einen Glykoprotein-Untereinheit-α(*gsuα*)-Promotor umfasst, der operabel mit einem Markergen verbunden ist.

11. Verfahren nach Anspruch 9, wobei die spezifische Wirkung eine Störung der Östrogenaktivität ist, wobei der Teleostenembryo ein transgener Teleostenembryo ist, der einen östrogensensitiven Promotor umfasst, der operabel mit einem Markergen verbunden ist.

12. Verfahren nach Anspruch 11, wobei der östrogensensitive Promotor Folgendes ist:
a) ein Aromatase-B-Promotor, und wobei der Teleostenembryo optional ein Zebrafischembryo oder ein Medakaembryo ist;
b) ein Choriogenin-Promotor, der optional ein Choriogenin-H-Promotor oder ein Choriogenin-L-Promotor ist, und wobei der Teleostenembryo optional ein Medakaembryo ist; oder
c) ein Vitellogenin-Promotor, und wobei der Teleostenembryo optional ein Zebrafischembryo oder ein Medakaembryo ist.

13. Verfahren nach Anspruch 9, wobei die spezifische Wirkung eine Störung der Androgenaktivität ist, wobei der Teleostenembryo ein transgener Teleostenembryo ist, der einen androgensensitiven Promotor umfasst, der operabel mit einem Markergen verbunden ist, wobei der androgensensitive Promotor optional ein *Spiggin-Promotor* ist und wobei der Teleostenembryo optional ein Medakaembryo oder ein Zebrafischembryo ist.

14. Verfahren nach Anspruch 9, wobei die spezifische Wirkung eine Störung der Schilddrüsenaktivität ist, wobei der Teleostenembryo ein transgener Teleostenembryo ist, der einen Schilddrüsenhormon(TH)-sensitiven Promotor umfasst, der operabel mit einem Markergen verbunden ist, wobei der TH-sensitive Promotor optional ein Schilddrüsen-stimulierender Hormon-Untereinheit-β(TSHβ)-Promotor ist und wobei der Teleostenembryo optional ein Medakaembryo oder ein Zebrafischembryo ist.

15. Verfahren nach Anspruch 9, wobei die spezifische Wirkung eine xenobiotische Wirkung ist, wobei der Teleostenembryo ein transgener Teleostenembryo ist, der einen xenobiotisch sensitiven Promotor umfasst, der operabel mit einem Markergen verbunden ist, wobei der xenobiotisch sensitive Promotor optional ein P450 1A-Promotor ist und wobei der Teleostenembryo optional ein Medakaembryo oder ein Zebrafischembryo ist.

16. Verfahren nach einem der Ansprüche 1 bis 15, wobei das Bestimmen, ob die Probe oder der Extrakt eine toxische Wirkung auf den Embryo ausübt, den Nachweis oder die Messung von Veränderungen in der Expression des Markergens umfasst.

17. Verfahren nach Anspruch 9, wobei die spezifische Wirkung eine xenobiotische Wirkung ist, wobei das Bestimmen, ob die Probe oder der Extrakt eine toxische Wirkung auf den Embryo ausübt, den Nachweis oder die Messung von Veränderungen der Ethoxyresorufin-O-Deethylase(EROD)-Aktivität umfasst und wobei der Teleostenembryo optional ein Medakaembryo oder ein Zebrafischembryo ist.

18. Verfahren nach Anspruch 9, wobei die spezifische Wirkung eine Kardiotoxizitätswirkung ist, wobei das Bestimmen, ob die Probe oder der Extrakt eine toxische Wirkung auf den Embryo ausübt, den Nachweis oder die Messung von Veränderungen der Herzentwicklung und/oder der Durchblutungsrate umfasst.

19. Verfahren nach Anspruch 18, wobei:
a) der Embryo einen BMP4-Promotor beherbergt, der operabel mit einem Markergen verbunden ist und, wobei der Nachweis oder die Messung von Veränderungen in der Herzentwicklung die Überwachung der Markergenexpression umfasst; oder
b) der Embryo einen Gata-1-Promotor beherbergt, der operabel mit einem Markergen verbunden ist, und wobei der Nachweis oder die Messung von Veränderungen der Durchblutungsrate die Überwachung der Markergenexpression umfasst.

20. Verfahren nach Anspruch 9, wobei die spezifische Wirkung eine Hepatotoxizitätswirkung ist und wobei das Bestimmen, ob die Probe oder der Extrakt eine toxische Wirkung auf den Embryo ausübt, den Nachweis oder die Messung von Veränderungen in der Leberentwicklung umfasst.

21. Verfahren nach einem der Ansprüche 10-16 und 19, wobei das Markergen Folgendes kodiert:
a) ein fluoreszierendes Protein, das optional ein grün fluoreszierendes Protein (GFP), ein cyan fluoreszierendes Protein (CFP), ein gelb fluoreszierendes Protein (YFP), ein rot fluoreszierendes Protein (dsRFP), Luciferase (Luc), Chloramphenicol-Acetyltransferase (CAT), 13-Galactosidase (LacZ) oder β-Glucuronidase (Gus) ist; oder
b) ein in einem kolorimetrischen Assay nachweisbares Enzym, wobei das Enzym optional eine Luciferase, Meerrettichperoxidase, β-Galactosidase, β-Glucuronidase, alkalische Phosphatase, Chloramphenicolacetyltransferase oder Alkoholdehydrogenase ist.

22. Verfahren nach einem der Ansprüche 10-16 und 19, wobei der Promotor von dem Teleostenembryo stammt.

23. Verfahren nach einem der Ansprüche 10-16 und 19, wobei der Promotor nicht von dem Teleostenembryo stammt, wobei optional:
a) der Teleostenembryo ein Zebrafischembryo ist und der Promotor von einem Medakafisch stammt, wobei der Medakafisch optional *Oryzias melastigma* oder *Oryzias latipes* ist; oder
b) der Teleostenembryo ein Medakaembryo ist und der Promotor von einem Zebrafisch stammt, wobei der Medakaembryo optional ein *Oryzias melastigma*-Embryo oder ein *Oryzias latipes*-Embryo ist.

24. Verfahren nach einem der Ansprüche 1 bis 23, wobei das Verfahren in einer Multiwell-Platte, optional einer 24-Well-Platte, 96-Well-Platte oder einer 384-Well-Platte, durchgeführt wird.

25. Verfahren nach einem der Ansprüche 1 bis 24, wobei mehr als eine Probe eines kosmetischen Produkts untersucht wird, wobei jede Probe optional in zweifacher oder dreifacher Ausführung untersucht wird.

26. Verfahren nach einem der Ansprüche 1 bis 24, wobei das Verfahren das Testen mehrerer Verdünnungen einer Probe oder eines Extrakts eines kosmetischen Produkts umfasst.

27. Verfahren nach einem der Ansprüche 1 bis 21, das eine beliebige Kombination der Verfahren der Ansprüche 24-26 umfasst.

## Revendications

1. Procédé de détermination de la toxicité totale dans un produit cosmétique, comprenant :
a) la combinaison d'un solvant et d'un échantillon du produit cosmétique pour obtenir un extrait de solvant avec une toxicité totale ;
b) la mise en contact d'un embryon téléostéen avec l'extrait de solvant de a) ; et
c) la détermination du fait que l'extrait exerce ou non un effet de toxicité sur l'embryon ;
dans lequel un effet de toxicité sur l'embryon représente une toxicité totale du produit cosmétique ; et dans lequel l'extrait est (i) un extrait de solvant organique ou (ii) un extrait de solvant organique et inorganique ;
dans lequel le solvant organique est du méthanol, DMSO, de l'acétonitrile ou acétone, et dans lequel le solvant inorganique est de l'eau ; dans lequel, lorsque l'échantillon du produit cosmétique est un liquide, le volume du solvant est d'environ 1,5 à environ 10 fois le volume de l'échantillon ; ou, lorsque l'échantillon du produit cosmétique est un solide ou semi-solide, le volume du solvant est d'environ 1,5 à environ 10 fois le poids de l'échantillon.

2. Procédé selon la revendication 1, dans lequel le produit cosmétique est un masque, un parfum, un antiperspirant, un déodorant, un dentifrice, un shampooing, une huile essentielle, un démaquillant, un produit de nettoyage, un colorant capillaire, un fond de teint, un anticernes, une crème solaire, une crème hydratante, une crème antirides, un fard à paupières, un crayon pour les yeux, un mascara, un fard à joue, un rouge à lèvres, un brillant à lèvre, un vernis à ongles, un sérum, une crème pour les yeux, une crème de nuit, une crème de jour, une crème BB, une lotion de nuit, une lotion de jour, une crème pour les mains, une crème pour le cou, ou une crème antivieillissement.

3. Procédé selon la revendication 1 ou la revendication 2, dans lequel le produit cosmétique est (i) un produit cosmétique hydromiscible ou (ii) un produit cosmétique non hydromiscible.

4. Procédé selon l'une quelconque des revendications 1 à 3, dans lequel l'extrait peut être obtenu par un processus comprenant (a) l'association d'un échantillon du produit cosmétique avec le solvant organique pour former un mélange, (b) le mélangeage du mélange, optionnellement par agitation vortex et/ ou sonification du mélange, (c) la séparation d'une phase contenant le solvant organique à partir du mélange, optionnellement en centrifugeant le mélange, et (d) la récupération de l'extrait, optionnellement dans lequel l'extrait est le surnageant ou une forme concentrée de celui-ci.

5. Procédé selon l'une quelconque des revendications 1 à 4, dans lequel les solvants organique et inorganique sont utilisés en un rapport de 3:7 à 7:3, optionnellement dans lequel le rapport est de 4:6 à 6:4.

6. Procédé selon l'une quelconque des revendications 1 à 5, comprenant en outre, avant l'étape (a), la préparation de l'extrait à partir de l'échantillon du produit cosmétique.

7. Procédé selon l'une quelconque des revendications 1 à 6, dans lequel le produit cosmétique est un produit cosmétique liquide hydromiscible et dans lequel l'étape (a) comprend la mise en contact d'un embryon téléostéen avec un échantillon du produit cosmétique.

8. Procédé selon l'une quelconque des revendications 1 à 7, dans lequel l'embryon téléostéen est :
a) un éleuthéroembryon ; et/ou
b) un embryon de médaka, optionnellement qui est un embryon de médaka transgénique ; un embryon de dard-perche, optionnellement qui est un embryon de dard-perche transgénique ; ou un embryon de tête-de-boule.

9. Procédé selon l'une quelconque des revendications 1 à 8, dans lequel l'effet de toxicité comprend un effet aigu, qui optionnellement comprend la mortalité, la malformation ou une association de celles-ci ; ou un effet spécifique.

10. Procédé selon la revendication 9, dans lequel l'effet spécifique est une interruption d'activité endocrinienne, qui est optionnellement une interruption d'activité estrogénique, une interruption d'activité androgénique ou une interruption d'activité thyroïdienne, optionnellement dans lequel l'embryon téléostéen est un embryon téléostéen transgénique comprenant ; un promoteur de sous-unité de glycoprotéine α (*gsuα*) fonctionnellement lié à un gène marqueur.

11. Procédé selon la revendication 9, dans lequel l'effet spécifique est une interruption d'activité estrogénique, dans lequel l'embryon téléostéen est un embryon téléostéen transgénique comprenant un promoteur à sensibilité estrogénique fonctionnellement lié à un gène marqueur.

12. Procédé selon la revendication 11, dans lequel le promoteur à sensibilité estrogénique est :
a) un promoteur d'aromatase B, et optionnellement dans lequel l'embryon téléostéen est un embryon de dard-perche ou un embryon de médaka ;
b) un promoteur de choriogénine, qui est optionnellement un promoteur de choriogénine H ou un promoteur de choriogénine L, et optionnellement dans lequel l'embryon téléostéen est un embryon de médaka ; ou
c) un promoteur de vitellogénine, et optionnellement dans lequel l'embryon téléostéen est un embryon de dard-perche ou un embryon de médaka.

13. Procédé selon la revendication 9, dans lequel l'effet spécifique est interruption d'activité androgénique, dans lequel
l'embryon téléostéen est un embryon téléostéen transgénique comprenant un promoteur à sensibilité androgénique fonctionnellement lié à un gène marqueur, optionnellement dans lequel le promoteur à sensibilité androgénique est un promoteur de *spiggin* et optionnellement dans lequel l'embryon téléostéen est un embryon de médaka ou un embryon de dard-perche.

14. Procédé selon la revendication 9, dans lequel l'effet spécifique est l'interruption d'activité thyroïdienne, dans lequel
l'embryon téléostéen est un embryon téléostéen transgénique comprenant un promoteur à sensibilité hormonale thyroïdienne (TH) fonctionnellement lié à un gène marqueur, optionnellement dans lequel le promoteur à sensibilité TH est un promoteur de sous-unité β hormonale à stimulation thyroïdienne (TSHβ), et optionnellement dans lequel l'embryon téléostéen est un embryon de médaka ou un embryon de dard-perche.

15. Procédé selon la revendication 9, dans lequel l'effet spécifique est un effet xénobiotique, dans lequel l'embryon téléostéen est un embryon téléostéen transgénique comprenant un promoteur à sensibilité xénobiotique fonctionnellement lié à un gène marqueur, optionnellement dans lequel le promoteur à sensibilité xénobiotique est un promoteur P450 1A, et optionnellement dans lequel l'embryon téléostéen est un embryon de médaka ou un embryon de dard-perche.

16. Procédé selon l'une quelconque des revendications 1 à 15, dans lequel la détermination du fait que l'échantillon ou l'extrait exerce ou non un effet de toxicité sur l'embryon comprend la détection ou la mesure de changements d'expression du gène marqueur.

17. Procédé de la revendication 9, dans lequel l'effet spécifique est un effet xénobiotique, dans lequel la détermination du fait que l'échantillon ou l'extrait exerce ou non un effet de toxicité sur l'embryon comprend la détection ou la mesure de changements d'activité éthoxyrésorufin-O-dééthylase (EROD), et optionnellement dans lequel l'embryon téléostéen est un embryon de médaka ou un embryon de dard-perche.

18. Procédé selon la revendication 9, dans lequel l'effet spécifique est un effet de cardiotoxicité, optionnellement dans lequel la détermination du fait que l'échantillon ou l'extrait exerce ou non un effet de toxicité sur l'embryon comprend la détection ou la mesure d'altérations de développement cardiaque et/ou de débit de circulation sanguine.

19. Procédé selon la revendication 18, dans lequel :
a) l'embryon a un promoteur BMP4 fonctionnellement lié à un gène marqueur et dans lequel la détection ou la mesure d'altérations de développement cardiaque comprenant la surveillance d'expression de gène marqueur ; ou
b) l'embryon a un promoteur gata1 fonctionnellement lié à un gène marqueur et dans lequel la détection ou la mesure d'altérations de débit de circulation sanguine comprend la surveillance d'expression de gène marqueur.

20. Procédé selon la revendication 9, dans lequel l'effet spécifique est un effet d'hépatotoxicité, et optionnellement dans lequel la détermination du fait que l'échantillon ou l'extrait exerce ou non un effet de toxicité sur l'embryon comprend la détection ou la mesure de changements de développement hépatique.

21. Procédé selon l'une quelconque des revendications 10 à 16 et 19, dans lequel le gène marqueur encode :
a) une protéine fluorescente, qui est optionnellement une protéine fluorescente verte (GFP), une protéine fluorescente cyan (CFP), une protéine fluorescente jaune (YFP), une protéine fluorescente rouge (dsRFP), de la luciférase (Luc), chloramphénicol acétyltransférase (CAT), 13-galactosidase (LacZ) ou β-glucuronidase (Gus) ; ou
b) un enzyme détectable dans une analyse colorimétrique, optionnellement dans lequel l'enzyme est une luciférase, peroxydase de raifort, β-galactosidase, β-glucuronidase, phosphatase alcaline, chloramphénicol acétyltransférase, ou alcool déshydrogénase.

22. Procédé selon l'une quelconque des revendications 10 à 16 et 19, dans lequel le promoteur est natif à l'embryon téléostéen.

23. Procédé selon l'une quelconque des revendications 10 à 16 et 19, dans lequel le promoteur n'est pas natif à l'embryon téléostéen, optionnellement dans lequel :
a) l'embryon téléostéen est un embryon de dard-perche et le promoteur est natif à un poisson médaka, dans lequel le poisson médaka est optionnellement *Oryzias melastigma* ou *Oryzias latipes* ; ou
b) l'embryon téléostéen est un embryon de médaka et le promoteur est natif à un dard-perche, dans lequel l'embryon de médaka est optionnellement un embryon d'*Oryzias melastigma* ou un embryon d'*Oryzias latipes.*

24. Procédé selon l'une quelconque des revendications 1 à 23, dans lequel le procédé est réalisé dans une plaque multipuits, optionnellement une plaque à 24 puits, une plaque à 96 puits ou une plaque à 384 puits.

25. Procédé selon l'une quelconque des revendications 1 à 24, dans lequel plus d'un échantillon de produit cosmétique est analysé, optionnellement dans lequel chaque échantillon est analysé en double ou en triple.

26. Procédé selon l'une quelconque des revendications 1 à 24, dans lequel le procédé comprend l'analyse de multiples dilutions d'un échantillon ou extrait de produit cosmétique.

27. Procédé selon l'une quelconque des revendications 1 à 21, qui comprend une quelconque association des procédés des revendications 24 à 26.
